**Europäisches Patentamt**

**European Patent Office**

**Office européen des brevets**

(11) Veröffentlichungsnummer: **0 195 354**
**A2**

## (12) EUROPÄISCHE PATENTANMELDUNG

(21) Anmeldenummer: **86103249.8**

(51) Int. Cl.⁴: **A61K 49/00**

(22) Anmeldetag: **11.03.86**

(30) Priorität: **20.03.85 DE 3510115**

(43) Veröffentlichungstag der Anmeldung:
**24.09.86 Patentblatt 86/39**

(84) Benannte Vertragsstaaten:
**AT BE CH DE FR GB IT LI LU NL SE**

(71) Anmelder: **Leopold & Co. Chem. pharm. Fabrik Gesellschaft m.b.H.**
**Hafnerstrasse 36**
**A-8055 Graz(AT)**
Anmelder: **Lentia Gesellschaft mit beschränkter Haftung**
**Arabellastrasse 4 Postfach 81 05 08**
**D-8000 München 81(DE)**

(72) Erfinder: **Hohlbrugger, Gero, Dr.**
**Moosmahdstrasse 14**
**A-6850 Dornbirn(AT)**
Erfinder: **Groke, Karl, Dr.**

**A-8063 Eggersdorf 86(AT)**

(74) Vertreter: **Allgeuer, Dorothea et al**
**Chemie Linz AG Patentabteilung St. Peter-Strasse 25**
**A-4020 Linz(AT)**

(54) **Wässrige Lösung zur urodynamischen Untersuchung der Harnblase und Verfahren zu deren Herstellung.**

(57) Wäßrige Lösung zur urodynamischen Untersuchung der Harnblase zur Diagnose von Entleerungsstörungen, welche pro Liter 50 -70 mmol Kaliumion enthält, auf einen durch Pufferzusatz stabilisierten pH-Wert von 4,7 -5,2 eingestellt ist und außerdem solche Mengen an harnüblichen Elektrolyten und/oder waserlöslichen, stabilen, niedermolekularen, organischen Substanzen enthält, daß die Lösung eine Osmolarität von 1000 bis 1360 mosmol/l Lösung aufweist.

EP 0 195 354 A2

Wäßrige Lösung zur urodynamischen Untersuchung der Harnblase und Verfahren zu deren Herstellung

Die vorliegende Erfindung betrifft eine wäßrige Lösung zur urodynamischen Untersuchung der Harnblase, die der Diagnose von Blasenentleerungsstörungen dient.

Solche Entleerungsstörungen der Harnblase können entweder in einer Areflexie bestehen, d.h. auch bei gefüllter Harnblase bleibt eine Muskelkontraktion, die zum Harndrang führt, aus, oder aber in einer Hyperreflexie der Blase, die sich in einem sehr häufigen Harndrang äußert. Für die Diagnose solcher Entleerungsstörungen unter Anwendung handelsüblicher Zystometer wurde gemäß Jonas U. und Thüroff J., Urodynamische Untersuchungen, Urologie in Klinik und Praxis, G. Thieme Verlag Stuttgart/New York, 1982, Seite 254, vorgeschlagen, die Harnblase mit Wasser, isotoner Kochsalzlösung oder aber $CO_2$ zu füllen und das Volumen zu bestimmen, bei dem der Harndrang ausgelöst wird. Diese Art der Untersuchung beruhte auf der Lehrmeinung, daß die Reizauslösung zur Blasenentleerung ausschließlich durch neurale Impulse erfolgt, wobei angenommen wurde, daß das Urothel dicht sei und keinen Stoffaustausch erlaube.

In der Praxis befriedigte diese Testmethode jedoch nicht, da bei einer Reihe von Patienten, die über Beschwerden klagten, keine Entleerungsstörung feststellbar war, sich jedoch häufig später doch eine Erkrankung der Harnblase herausstellte.

Auf der anderen Seite wurde von Fellows G.J. und Marshall D.H. in Invest. Urol. 9, 339, 1972 bereits darauf hingewiesen, daß das Urothel durchaus einen Stoffaustausch mit dem Blut unter bestimmten Bedingungen zuläßt und dabei ein Austritt nicht nur von Wasser, sondern auch von Ionen, möglich ist, wobei dieser Stoffaustausch bei den meisten Erkrankungen der Harnblase noch zunimmt. Es wurde auch schon im Tierversuch beobachtet, daß Kalium Kontraktionen des Blasenmuskels von Schweinen bewirken kann. (V. Duyl W.A. und Collsaet B.L.R.A., Urology 20, 53, 1982)

Der vorliegenden Erfindung liegt die Aufgabe zugrunde, eine Testlösung für die urodynamische Untersuchung der Harnblase zu schaffen, die ein sicheres Erkennen von Entleerungsstörungen gestattet.

Der Lösung dieser Aufgabe gemäß vorliegender Erfindung liegt die überraschende Erkenntnis zugrunde, daß die Auslösung des Harndranges sehr stark von der Zusammensetzung des Harns bestimmt wird, wobei Kaliumgehalt, pH-Wert und Osmolarität den Harndrang steuern, sodaß bei konzentriertem Harn, der z. B. bezüglich seines Kaliumgehalts gegenüber dem Blut stark angereichert ist, und eine hohe Osmolarität besitzt, die Kontraktion der Harnblasenmuskulatur, die man als den die Blasenentleerung durchführenden Vorgang ansprechen kann, schon bei wesentlich geringerem Füllvolumen der Blase ausgelöst wird, als dies bei wesentlich verdünnterem Harn der Fall ist. Als weiterer auslösender Faktor erwies sich das H-Ion, das bis zu einem gewissen Grad das K-Ion intrazellulär vertreten kann.

Eine hohe Konzentration an K-Ionen und H-Ionen sowie eine Hyperosmolarität können daher eine Kontraktion der unter dem Urothel liegenden Muskulatur bewirken, ohne daß neurale Impulse vom ZNS her erfolgen.

Auf dieser Erkenntnis aufbauend konnte überraschenderweise gefunden werden, daß eine wäßrige Lösung für die urodynamische Untersuchung der Harnblase dann verläßliche Diagnosen von Entleerungsstörungen, und zwar sowohl einer Areflexie als auch einer Hyperreflexie ermöglicht, wenn sie hinsichtlich Kaliumgehalt und Osmolarität einem konzentrierten Harn entspricht und ihr pH-Wert im Bereich eines sauren Harns stabil gehalten wird. Eine solche Lösung bewirkt verläßlich eine für die untersuchte Harnblase frühe Auslösung des Harndranges, sodaß die damit erhaltenen Werte weit mehr der physiologischen Standortsituation der Blasenfunktion gerecht werden, als dies bei der bisherigen Diagnosemethode der Fall war. Das bedeutet, daß Areflexien der Blase nur dort ermittelt werden, wo solche tatsächlich bestehen und nicht auch in Fällen, bei denen die Blase für Wasser ein sehr großes Volumen aufweist, und ebenso, daß eine Hyperreflexie, beispielsweise verursacht durch eine erhöhte Durchlässigkeit des Urothels, nicht kaschiert wird.

Gegenstand der vorliegenden Erfindung ist demnach eine wäßrige Lösung zur urodynamischen Untersuchung der Harnblase, die dadurch gekennzeichnet ist, daß sie einen Gehalt an Kaliumionen von 50 bis 70 mmol/Liter und einen mit Hilfe eines Gehaltes an einer Puffersubstanz stabilisierten pH-Wert von 4,7 -5,2 aufweist und durch einen Gehalt an harnüblichen Elektrolyten und/oder wasserlöslichen, niedermolekularen, in wäßriger Lösung stabilen, blasenverträglichen, organischen Substanzen auf eine Osmolarität von etwa 1000 -1360 mosmol/l Lösung eingestellt ist.

Die Wahl der erfindungsgemäßen Konzentration an K-Ionen beruht darauf, daß die Konzentration an K-Ionen in konzentriertem Harn meist Werte um 60 mmol/l aufweist, sodaß der erfindungsgemäße Wert der physiologischen Bedingung der frühen Auslösung des Harndranges entspricht. Bei Einstellung des pH-Wertes von 4,7 -5,2 vorzugsweise von 5,0 ist zu beachten, daß die Harnblase bei Vorliegen eines sauren Harnes bestrebt ist, durch Austritt von Wasser aus der Blutbahn durch das Urothel den pH-Wert zu erhöhen, d. h. daß der saure pH bei Verweilen der Testlösung in der Harnblase, besonders bei stark durchlässigem Urothel, nicht erhalten bleiben würde und Fehldiagnosen die Folge wären. Daher ist es wesentlich, daß die erfindungsgemäße Lösung Puffersubstanzen enthält, die die Stabilisierung des pH-Wertes im sauren Bereich bewirken.

Bei der Auswahl von niedermolekularen und damit osmotisch wirksamen Substanzen, die neben Elektrolyten der Einstellung der erfindungsgemäß zu wählenden Osmolarität dienen, ist zu beachten, daß sich diese Substanzen in der schwach sauren wäßrigen Lösung nicht zersetzen. Harnstoff, der für die Osmolarität von Harn weitgehend bestimmend ist, ist hierfür wegen seiner Instabilität nicht geeignet. Ferner sind auch alle jene Substanzen auszuschließen, die auf Grund eines stark sauren oder alkalischen Eigen-pH-Wertes den pH-Wert der Lösung verschieben würden und schließlich sind auch Substanzen nicht geeignet, die die Blasentätigkeit beeinflussen, sei es, daß sie zu Reizerscheinungen führen oder auch die Blasentätigkeit hemmen. Als sehr zweckmäßig hat sich die Verwendung von wasserlöslichen Polyhydroxyverbindungen erwiesen, wobei sich Zuckeralkohole wie insbesondere Sorbit, Xylit und Mannit, Glucose und Fructose sowie Glycerin besonders bewährt haben. Die Zuckeralkohole Sorbit und Mannit sowie Glycerin sind dabei bevorzugt, Sorbit ist besonders bevorzugt. Häufig, vor allem im Falle der Verwendung von Monosacchariden als Substanzen zur Einstellung der Osmolarität ist es zweckmäßig, der Lösung eine geringe Menge an desinfiszierenden Mitteln zuzusetzen, da diese Zucker Nährstoffe für Bakterien darstellen und so in Fällen von Blaseninfektionen unter Umständen zu einer Vermehrung der Bakterien, die diese verursacht haben, beitragen und somit zu einer Verschlechterung des Zustandes des Patienten führen könnten. Als Beispiel für Desinfektionsmittel, die sich sehr bewährt haben, sind Benzalkoniumchlorid oder vor allem die Ester der 4-Hydroxybenzoesäure zu nennen, die allein oder in Mischung eingesetzt werden können.

Ferner hat sich auch der Zusatz stabilisierender Zusätze wie Antioxidanten in manchen Fällen bewährt, vor allem dann, wenn als niedermolekulare organische Substanzen zur Einstellung der Osmolarität reduzierende Zucker eingesetzt werden. Als soche sind z. B. Sulfite oder Pyrosulfite zu nennen.

Außer den Polyhydroxyverbindungen, die meist in größeren Mengen, z. B. in Mengen von etwa 100 -170 g/Liter Lösung zugesetzt werden können, ist es auch möglich, stabile, harngängige Substanzen wie etwa Kreatinin zur Einstellung der erfindungsgemäß geforderten Osmolarität mit heranzuziehen.

Da die erfindungsgemäße Lösung in ihrer Beschaffenheit möglichst der Zusammensetzung des Harns angepaßt sein soll, hat es sich bewährt, für die Einstellung der Osmolarität außer dem vorgeschriebenen Gehalt an K-Ionen und außer niedermolekularen organischen Substanzen auch andere Elektrolyte, wie sie im Harn üblicherweise

vorkommen, heranzuziehen. In erster Linie dienen hier wasserlösliche Salze von Natrium, Calcium und Magnesium, vorzugsweise in einer Menge und Zusammensetzung, die für den Harn typisch ist. Das heißt, daß größere Mengen an Natriumsalzen, beispielsweise die 2 -4fache molare Menge bezogen auf den vorhandenen Gehalt an Kaliumsalzen eingesetzt werden können, während die molaren Mengen an Ca-und Mg-Salzen zweckmäßig nur einen Bruchteil der vorhandenen molaren Mengen an Alkalimetallsalzen ausmachen sollen.

Die Salze des Kaliums und Natriums sind vorzugsweise Chloride und Sulfate, während Calcium bevorzugt als Chlorid, Magnesium als Chlorid und als Sulfat eingesetzt werden.

Da als Puffersubstanz in erster Linie Phosphate, z. B. solche des Natriums oder Kaliums zu empfehlen sind, ist es zweckmäßig, außerdem komplexierende Substanzen wie z. B. Salze der Äthylendiamintetraessigsäure zuzusetzen, um eine Niederschlagsbildung mit vorhandenem Ca-und/oder Mg-Ionen zu vermeiden.

Besonders bewährt hat sich erfindungsgemäß eine wäßrige Lösung, die pro Liter folgende Bestandteile enthält:

als Kationen

| | |
|---|---|
| K-Ion | 50 - 70 mmol |
| Na-Ion | 100 - 140 mmol |
| Ca-Ion | 1,5 - 4 mmol |
| Mg-Ion | 5 - 10 mmol |

als Anionen:

| | |
|---|---|
| Chlorid | 120 - 145 mmol |
| Sulfat | 10 - 20 mmol |

als Puffersubstanz:

| | |
|---|---|
| Phosphat | 40 - 65 mmol |

als wasserlösliche organische Substanz:

| | |
|---|---|
| Kreatinin | 0 - 2,5 g |

und Sorbit, Mannit oder Glycerin in einer Menge, daß sich zusammen mit den übrigen Bestandteilen der Lösung eine Osmolarität derselben von 1000 - 1360, vorzugsweise von 1000 - 1300 mosmol/l ergibt.

Zweckmäßig sind auch eine geringe Menge eines Komplexbildners und geringe Mengen desinfizierender Zusätze zugegen.

Besonders bevorzugt ist in solchen Lösungen pro Liter ein Gehalt an Kaliumion von 60 -70 mmol, ein solcher an Natriumion von 125 -140 mmol, an Calciumion von 2,5 mmol, an Magnesiumion von 7,5 mmol und die Gegenwart von Soribt als wasserlösliche organische Substanz in einer Menge von 120 -160 g, wobei die Lösung außerdem auch Kreatinin enthalten kann.

Der pH-Wert der Lösung ist vorzugsweise auf 5 eingestellt.

Mit der erfindungsgemäßen Lösung können völlig reproduzierbare, gleichmäßige Testergebnisse erhalten werden, sodaß sie sich für die Diagnose von Blasenfunktionsstörungen hervorragend eignet.

Vergleicht man die mit der erfindungsgemäßen Lösung unter Anwendung eines handelsüblichen Zystometers (siehe z. B. Urodynamische Untersuchungen, G. Thieme Verlag 1982, S. 254 ff) erhaltenen Werte für die Blasenvolumina, bei denen der Harndrang ausgelöst wird mit solchen, die bei Füllung der Harnblase mit Wasser erreicht werden, so zeigt sich, daß die mit Wasser erhaltenen Werte bis zu 80 % größer sein können und damit vorhandene Hyperrefle-

xien unter Umständen völlig verschleiern. Die erfindungsgemäße Lösung bewirkt signifikant höhere Blasenbinnendruckamplituden und beeinflußt die Sensibilität bzw. das Dranggefühl im Sinne einer früheren Auslösung.

Zur Herstellung der erfindungsgemäßen Lösung werden die Elektrolyte und/oder niedermolekularen Substanzen mit Ausnahme des Puffers in der nötigen Menge zur Erzielung einer Osmolarität innerhalb der erfindungsgemäßen Grenze in etwa der gewünschten Wassermenge, die sich auf einer Temperatur von 40 -80°C befindet, vollständig aufgelöst, wobei bei der Bemessung der Menge an Kaliumsalzen darauf Bedacht zu nehmen ist, ob der später zu erfolgende Pufferzusatz Kalium enthält oder nicht. Nach Einstellung des pH-Wertes, zu der meist eine Säure benötigt wird, deren Anion zweckmäßig den in der Lösung vorhandenen Anionen entspricht, wird die Puffersubstanz zugegeben und somit der pH-Wert stabilisiert. Anschließend wird die Lösung gegebenenfalls steril filtriert, gegebenenfalls mit Wasser zu Injektionszwecken auf das gewünschte Endvolumen gebracht und in üblicher Weise keimfrei gemacht.

Für den Gebrauch ist es besonders zweckmäßig, die erfindungsgemäße Lösung in flexible Beutel, z. B aus PVC, abzufüllen. Natürlich sind aber auch Glasgefäße als Behälter geeignet.

In manchen Fällen, vor allem dort, wo ein langer Transportweg oder eine größere Lagerhaltung vorgesehen ist, hat es sich bewährt, die fertige erfindungsgemäße Lösung zu lyophilisieren und das Lyophisilat in den Handel zu bringen. Dieses wird dann an Ort und Stelle vor der Verwendung in sterilem Wasser aufgelöst.

Beispiel 1:

897 g Wasser für Injektionen einer Temperatur von 20°C werden auf 60°C vorgewärmt, unter Bewegung mit 0,37 g $CaCl_2 . 2 H_2O$, 0,75 g KCl, 1,525 g $MgCl_2 . 6 H_2O$, 5,5 g NaCl, 5,32 g $Na_2SO_4 . 10H_2O$, 150 g Sorbit, 2,2 g Kreatinin, 0,1 g Äthylendiamintetraessigsäure-Dinatriumsalz sowie zu Desinfektionszwecken mit 0,15 g p-Hydroxy-benzoesäuremethylester, 0,025 g p-Hydroxybenzoesäureäthylester und 0,025 g p-Hydroxybenzoesäurepropylester versetzt und so lange gemischt, bis eine völlige Auflösung der Bestandteile erzielt ist. Anschließend wird die Lösung durch Zugabe von 0,6 g konzentrierter Salzsäure auf einen pH-Wert von 5,0 eingestellt, worauf als Puffersubstanz 6,8 g Kalium-dihydrogenphosphat zugesetzt werden und das Volumen exakt auf 1 l eingestellt wird.

Dabei resultiert eine Lösung, die pro Liter 128 mmol Natriumion, 60 mmol Kaliumion, 2,5 mmol Calciumion, 7,5 mmol Magnesiumion, 132 mmol Chloridion, 50 mmol H$_2$PO$_4$-lon und 16,5 mmol Sulfation enthält sowie eine Osmolarität von 1240 mosmol aufweist.

Beispiel 2:

Zur Herstellung einer erfindungsgemäßen Lösung etwa gleicher Osmolarität wie in Beispiel 1, jedoch ohne einen Gehalt an Kreatinin werden wie in Beispiel 1 beschrieben 0,370 g $CaCl_2.2H_2O$, 0,750 g KCl, 1,525 $MgCl_2.6H_2O$, 6,250 g NaCl, 5,320 g $Na_2SO_4.10 H_2O$, 150 g Sorbit sowie der Komplexbildner und die desinfizierenden Zusätze wie in Beispiel 1 in vorgewärmtem Wasser gelöst und wie in Beispiel 1 weiterverarbeitet.

Der Gehalt an Natriumion in der Lösung hat sich dadurch gegenüber Beispiel 1 auf 140,0 mmol/l, jener an Chloridion auf 144,20 mmol/l erhöht. Die Osmolarität der Lösung beträgt 1247 mosmol/l.

Beispiel 3:

- Die in Beispiel 1 angegebenen Bestandteile, jedoch mit dem Unterschied, daß anstelle von 150 g Sorbit 150 g Mannit eingesetzt werden, werden wie in Beispiel 1 angegeben zu 1 Liter Lösung verarbeitet. Der pH-Wert derselben beträgt 5,0, die Osmolarität 1240 mosmol/l.

Beispiel 4:

Die in Beispiel 1 angegebenen Bestandteile, jedoch mit dem Unterschied, daß als wasserlösliche organische Substanz anstelle von Sorbit und Kreatinin 76,0 g Glycerin eingesetzt werden, werden wie in Beispiel 1 zu 1 Liter Lösung verarbeitet. Der pH-Wert der Lösung beträgt 5,0, die Osmolarität 1224 mosmol/l.

Beispiel 5:

Die in Beispiel 1 genannten Bestandteile, jedoch mit dem Unterschied, daß NaCl in einer Menge von 5,080 g und die Puffersubstanz zur Erhöhung der Pufferkapazität in einer Menge von 8,165 g eingesetzt wird, werden wie in Beispiel 1 beschrieben zu 1 Liter einer Lösung verarbeitet, die den gleichen pH-Wert wie die Lösung gemäß Beispiel 1 und eine Osmolarität von 1227 mosmol/l besitzt. Der Gehalt an Natriumion wurde also gegenüber Beispiel 1 auf 120,0 mmol/l jener an Chlorid auf 124,20 mmol/l erniedrigt, der Phosphatgehalt auf 60 mmol und damit der Gehalt an Kaliumion auf 70 mmol/l erhöht.

Für die Anwendung werden diese Lösungen nach Sterilfiltration in Portionen von 4500 ml in Beutel aus Polyvinylchlorid abgefüllt und 50 Minuten bei 115°C sterilisiert. Sie sind bei Lagerung unter 20°C völlig stabil und können als Testlösung in jedem handelsüblichen Zystometer eingesetzt werden.

**Ansprüche**

1. Wäßrige Lösung zur urodynamischen Untersuchung der Harnblase, dadurch gekennzeichnet, daß sie einen Gehalt an Kaliumionen von 50 -70 mmol/Liter und einen mit Hilfe eines Gehaltes an einer Puffersubstanz stabilisierten pH-Wert von 4,7 -5,2 aufweist und durch einen Gehalt an harnüblichen Elektrolyten und/oder wasserlöslichen, niedermolekularen, in wäßriger Lösung stabilen, blasenverträglichen organischen Substanzen auf eine Osmolarität von etwa 1000 -1360 mosmol/l Lösung eingestellt ist.

2. Lösung nach Anspruch 1, dadurch gekennzeichnet, daß sie als Elektrolyte neben Kaliumsalzen Salze von Na, Ca und Mg in für Harn typischer Menge und Zusammensetzung enthält.

3. Lösung nach den Ansprüchen 1 und 2, dadurch gekennzeichnet, daß als wesentlicher Bestandteil zur Einstellung der Osmolarität ein Zuckeralkohol, Glucose, Fructose, Glyzerin oder Mischungen dieser Verbindungen eingesetzt werden.

4. Lösung nach den Ansprüchen 1 bis 3, dadurch gekennzeichnet, daß sie blasenverträgliche stabilisierende und desinfizierende Zusätze enthält.

5. Lösung nach den Ansprüchen 1 bis 5, dadurch gekennzeichnet, daß sie pro Liter neben den 50 bis 70 mmol Kaliumion als weitere Kationen 100 -140 mmol Natriumion,